# FASCICULE DE BREVET EUROPEEN

(11) **EP 1 185 209 B1**
(45) Date de publication et mention de la délivrance du brevet: **14.09.2005**
(21) Numéro de dépôt: 00945974.4
(22) Date de dépôt: 14.06.2000
(51) Int. Cl.: A61B 17/70

(54) **IMPLANT POUR DISPOSITIF D'OSTEOSYNTHESE NOTAMMENT DU RACHIS**
IMPLANTAT FÜR OSTEOSYNTHESEVORRICHTUNG, INSBESONDERE FÜR DIE WIRBELSÄULE
IMPLANT FOR OSTEOSYNTHESIS DEVICE IN PARTICULAR OF THE BACKBONE

(30) Priorité: 14.06.1999 FR 9907687
(43) Date de publication de la demande: 13.03.2002
(73) Titulaire: SCIENT'X, 78284 Guyancourt (FR)
(72) Inventeur: CARLI, Olivier, CH-1207 Genève (CH)
(74) Mandataire: Thibault, Jean-Marc
(86) Numéro de dépôt international: PCT/FR2000/001644
(87) Numéro de publication internationale: WO 2000/076413

(56) Documents cités:
- EP-A- 0 836 835
- WO-A-98/34554
- WO-A-98/41159
- DE-A- 4 425 357

## Description

La présente invention concerne le domaine technique de l'ostéosynthèse, notamment du rachis, et elle vise plus précisément un implant comportant des vis d'ancrage dans les vertèbres, conçues pour permettre d'orienter angulairement une tige de liaison s'étendant en relation desdites vertèbres en vue de les maintenir immobilisées pendant une phase de fusion osseuse.

### TECHNIQUE ANTERIEURE :

Différents systèmes ont été développés pour corriger et stabiliser le rachis et pour faciliter la fusion osseuse à différents niveaux du rachis. Selon l'un des systèmes, une tige apte à être cintrée est disposée le long du rachis, tout en étant maintenue en position par des vis implantées dans les vertèbres pour suivre la courbure de la région du rachis appareillée. Ainsi, pour respecter l'anatomie du rachis, la tige de liaison doit être conformée pour présenter des angulations importantes, notamment pour son montage en relation des vertèbres lombaires et sacrée.

Pour autoriser de telles conformations de la tige tout en assurant son blocage efficace par rapport aux vis d'ancrage, il a été proposé d'équiper les vis d'ancrage, d'une articulation sphérique pour recevoir la tige de liaison, de manière à autoriser une angulation relative adaptative entre la vis d'ancrage et la tige de liaison

Ainsi, par exemple, le brevet **EP 0 614 649** décrit un implant pour dispositif d'ostéosynthèse comportant un corps de fixation conformé sous la forme d'une douille dans laquelle est aménagé un canal de réception pour une tige de liaison. Ce corps de fixation est aménagé pour présenter un logement de réception pour une tête d'une vis d'ancrage afin de déterminer une articulation sphérique entre la vis d'ancrage et le corps de fixation. Cet implant comporte également une bague de positionnement destinée à être interposée entre la tête de la vis d'ancrage et la tige de liaison. Cet implant comporte également un système du type écrou pour l'assemblage de la tige de liaison sur le corps de fixation. Un tel système comporte un écrou vissé sur les parois externes du corps de fixation, tandis qu'un bouchon fileté est vissé à l'intérieur du corps de fixation. Le vissage d'un tel dispositif d'assemblage permet d'assurer le blocage par serrage, d'une part, de la tige de liaison entre le bouchon fileté et la bague de positionnement et, d'autre part, de la vis d'ancrage entre la bague de positionnement et le corps de fixation.

Il doit être considéré qu'un tel implant est constitué par plusieurs pièces avec lesquelles il convient d'effectuer des assemblages intermédiaires en per opératoire. Il s'ensuit une difficulté pour son montage et un temps de mise en place relativement important.

Dans le même sens, le document WO98/34554 décrit un implant analogue comportant une bague de positionnement pourvue de stries destinées à coopérer avec des crans aménagés sur la tête de la vis d'ancrage.

De même, le document EP 0 836 835 décrit selon une variante de réalisation, un implant pour dispositif d'ostéosynthèse comportant un corps de fixation pour la réception d'une tige de liaison. Ce corps comporte une douille déformable destinées après la mise en place de la vis d'ancrage, à recevoir la tête de la tête de la vis d'ancrage.

Le document **DE 44 25 357** état de la technique la plus proche décrit également un implant pour dispositif d'ostéosynthèse comportant un premier ensemble comprenant un corps de fixation aménagé pour présenter un logement de réception pour une tête d'une vis d'ancrage, afin de déterminer une articulation sphérique entre la vis d'ancrage et le corps de fixation. Ce premier ensemble comprend également une bague de positionnement destinée à être interposée entre la tête de la vis d'ancrage et une tige de liaison. Cet implant comporte également un deuxième ensemble comportant un système du type écrou assurant l'assemblage de la tige de liaison sur le corps de fixation. Un tel implant ne permet pas d'assurer une liaison efficace entre la vis d'ancrage et le corps de fixation entraînant une instabilité de la tige de liaison par rapport à la vis d'ancrage.

### EXPOSE DE L'INVENTION :

L'objet de l'invention vise donc à remédier aux inconvénients des implants de l'art antérieur en proposant un implant pour un dispositif d'ostéosynthèse du rachis comportant une vis d'ancrage osseux équipée d'une articulation sphérique pour recevoir une tige de liaison, un tel implant étant conçu pour être rapidement et facilement mis en place, tout en étant adapté pour permettre une liaison efficace et durable entre la tige de liaison et la vis d'ancrage osseux.

Pour atteindre un tel objectif, cet implant pour dispositif d'ostéosynthèse, notamment du rachis comporte :
- un premier ensemble comportant :
   - un corps de fixation pour une tige de liaison, ledit corps étant aménagé pour présenter un logement de réception pour une tête d'une vis d'ancrage, afin de déterminer une articulation sphérique entre la vis d'ancrage et le corps de fixation,
   - une bague de positionnement destinée à être interposée entre la tête de la vis d'ancrage et la tige de liaison,
- et un deuxième ensemble comportant un système du type écrou, pour l'assemblage de la tige de liaison sur le corps de fixation.

Selon l'invention :
- le premier ensemble comporte la bague de positionnement qui est guidée en déplacement linéaire limitée dans le corps de fixation pour autoriser, en l'absence de la tige de liaison, une libre rotation entre le corps et la vis d'ancrage,
- le deuxième ensemble comporte un système du type écrou adapté pour permettre lors de son vissage sur le corps, l'appui sur la tige de liaison et le déplacement linéaire de la bague de positionnement, en vue d'assurer le blocage par serrage, d'une part, de la tige de liaison entre ledit système et la bague de positionnement et, d'autre part, de la vis d'ancrage entre la bague de positionnement et le corps de fixation.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

### BREVE DESCRIPTION DES DESSINS :

La **fig. 1** est une vue en perspective montrant un implant complet recevant une tige de liaison intervertébrale.
La **fig. 2** est une vue en perspective d'un premier ensemble formant l'implant conforme à l'invention.
La **fig. 3** est une vue en coupe-élévation du premier ensemble, prise sensiblement selon les lignes III-III de la **fig. 2.**
La **fig. 4** est une vue en coupe-élévation d'un implant conforme à l'invention prise sensiblement selon les lignes IV-IV de la **fig. 1.**
La **fig. 5** est une vue en perspective d'un second ensemble formant l'implant conforme à l'invention.
La **fig. 6** est une vue en coupe d'un second ensemble, prise sensiblement selon les lignes VI-VI de la **fig. 5.**

### MEILLEURE MANIERE DE REALISER L'INVENTION :

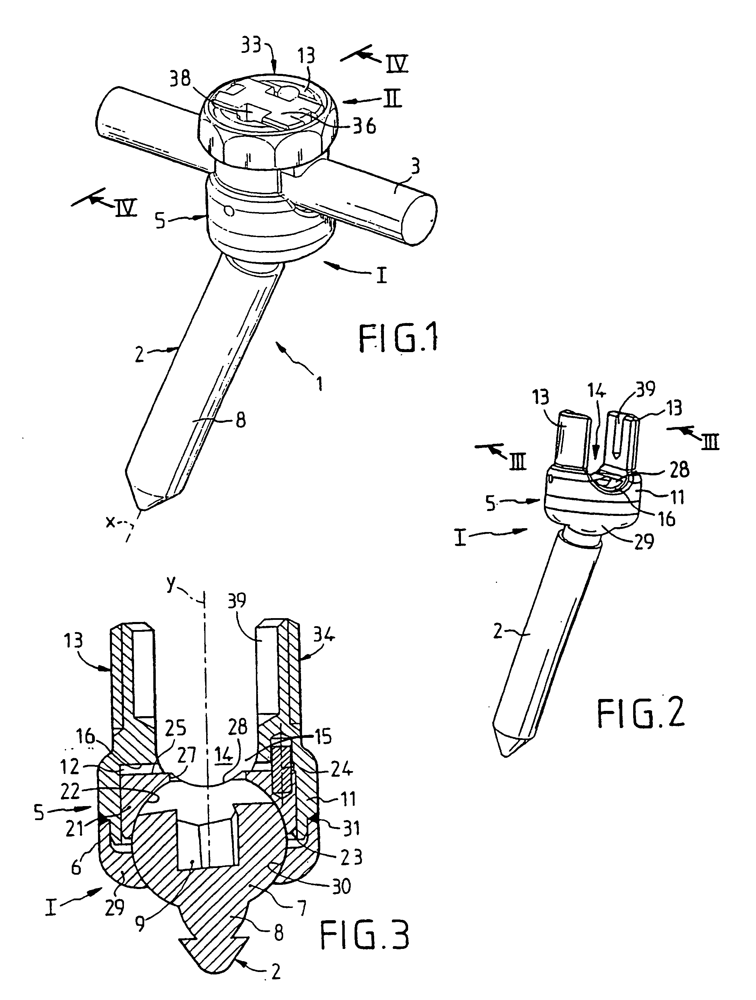

L'implant **1** représenté sur la **fig. 1** est destiné à un dispositif d'ostéosynthèse non représenté, notamment du rachis. Conformément à l'invention, cet implant **1** est constitué d'un premier ensemble **I** comprenant, notamment, une vis d'ancrage osseux **2** et un deuxième ensemble **II** conçu pour assurer l'assemblage d'une tige de liaison intervertébrale **3** par rapport à la vis d'ancrage **2.**

Tel que cela apparait plus précisément aux **fig. 2** et **3,** le premier ensemble **I** comporte un corps de fixation **5** aménagé pour présenter un logement **6** de réception pour la tête **7** de la vis d'ancrage **2.** qui s'étend à l'extrémité d'une tige d'ancrage filetée **8** d'axe longitudinal x De manière classique, la tête **7** de la vis d'ancrage **2** présente une forme generale sphérique tronquée à son sommet qui est pourvue d'un trou borgne **9** de section polygonale pour permettre la rotation de la vis d'ancrage **2** à l'aide d'un outil de vissage non représenté mais connu en soi.

Dans l'exemple illustré, le corps de fixation **5** comporte une tête **11** constituée sous la forme d'une douille d'axe longitudinal y, à l'intérieur de laquelle est aménagée une cavité **12** centrée sur l'axe longitudinal y. Selon un exemple préféré de réalisation, deux parois ou branches latérales **13** diamétralement opposées, s'élèvent à partir de la tête de fixation **11** pour définir entre elles un canal **14** de réception de la tige de liaison **3.** La cavité **12** s'ouvre dans le canal **14** entre les branches latérales 13, par l'intermédiaire d'un orifice **15** aménagé dans le fond **16** de la cavité **12**. Le canal de réception **14** s'ouvre de part et d'autre de la tête **5,** selon une direction perpendiculaire au plan de symétrie diamétrale passant par les branches latérales **12.** De préférence, le canal de réception **14** est partiellement aménagé dans la partie supérieure de la tête de fixation **11** par l'intermédiaire d'une entaille **16** de profil semi-circulaire pour permettre l'insertion partielle de la tige de liaison **3** qui présente classiquement une section transversale circulaire.

Le premier ensemble **I** comporte également une bague de positionnement **21** destinée à être interposée entre la tête **7** de la vis d'ancrage et la tige de liaison **3.** Cette bague de positionnement **21** est montée à l'intérieur de la cavité **12** et présente un alésage central **22** de forme partiellement sphérique débouchant sur une première face transversale **23** pour coopérer avec la partie supérieure de la tête **7** de la vis d'ancrage. Bien entendu, le plus grand diamètre de l'alésage central **22** est inférieur au diamètre de la tête **7** de la vis d'ancrage. La bague de positionnement **21** possède un déplacement limité selon l'axe de symétrie y du corps **5,** entre le fond **16** de la cavité **12** et la tête **7** de la vis d'ancrage. La bague de positionnement **21** est guidée en déplacement linéaire limitée, selon l'axe longitudinal y. La bague **21** est guidée en déplacement linéaire par l'intermédiaire d'un pion de guidage **24** interposé entre le corps de fixation **5** et la bague de positionnement **21.** Par exemple, le pion de guidage **24** est engagé dans des alésages borgnes aménagés dans le fond 16 de la cavité 12 et dans une deuxième face transversale **25** de la bague de positionnement s'étendant en regard du fond **16** de la cavité.

Il est à noter que l'alésage central **22** de la bague de positionnement **21** débouche par une ouverture de passage **27,** sur la deuxième face transversale **25,** pour communiquer avec l'orifice **15** aménagé dans la tête **11,** de manière à autoriser l'accès, pour l'outil de vissage, au trou borgne **9** de la vis d'ancrage. De préférence, la deuxième face transversale **25** de la bague de positionnement **21** présente une surface **28** concave congruente à la tige de liaison **3.** Cette surface concave **28** forme ainsi un genre de berceau venant dans le prolongement de l'entaille **16** pour délimiter en partie le canal de réception **14** de la tige de liaison **3.** Il est à noter que cette face concave **28** se trouve automatiquement positionnée dans le prolongement des entailles **16,** en vue de recevoir la tige de liaison **3,** dans la mesure où la bague de positionnement **21** est guidée en déplacement linéaire selon l'axe longitudinal y.

Tel que cela ressort plus précisément de la **fig. 3,** la vis d'ancrage **2** est maintenue assemblée sur le corps de fixation **5** à l'aide d'une coupelle d'obturation **29** fixée sur la tête de fixation **11.** Cette coupelle d'obturation **29** possède un alésage central **30** de forme partiellement sphérique complémentaire du profil de la partie inférieure de la tête **7** de la vis d'ancrage. L'alésage central **30** possède un plus grand diamètre qui, bien entendu, est inférieur au diamètre de la tête **7** de la vis d'ancrage. Dans l'exemple illustré, la coupelle d'obturation **29** est fixée à la tête de fixation **11** par l'intermédiaire d'un cordon périphérique de soudure **31.** La vis d'ancrage **2** se trouve ainsi montée sur le corps de fixation **5** par l'intermédiaire d'une articulation sphérique permettant d'obtenir, entre le corps de fixation **5** et la vis d'ancrage **2,** un débattement angulaire s'établissant dans un cône. La tête **7** de la vis d'ancrage **2** coopère ainsi avec les alésages internes **22, 30** respectivement de la bague de positionnement **21** et de la coupelle d'obturation **29,** pour délimiter ensemble le logement **6** de guidage en rotation de la tête **7** de la vis d'ancrage.

Le montage de cet ensemble **I** découle directement de la description qui précède. La tête de fixation **11** est destinée à recevoir dans la cavité **12,** la bague de positionnement **21,** en assurant l'engagement du pion de guidage **24** entre la bague de positionnement **21** et la tête de fixation **11.** La coupelle d'obturation **29** est introduite par son alésage interne **30,** à partir de l'extrémité filetée **8** de la vis d'ancrage **2,** jusqu'au niveau de la tête **7.** La tête **7** de la vis d'ancrage **2** est introduite à l'intérieur de l'alésage interne **22** de la bague de positionnement **21.** La coupelle d'obturation **29** qui est traversée par la vis d'ancrage **2** est fixée sur la tête de fixation **11** par une soudure dans l'exemple illustré. Il est à noter qu'en l'absence de la tige de liaison **3,** la bague de positionnement **21** possède une liberté de déplacement linéaire limité autorisant une rotation relative de la tête **7** de la vis d'ancrage **2** par rapport au corps de fixation **5.**

Il doit être considéré que la vis d'ancrage **2** se trouve assemblée au corps de fixation **5** préalablement à son utilisation. Ainsi, l'ensemble **I** se présente sous la forme d'une pièce unitaire adaptée pour recevoir directement une tige de liaison **3** qui est fixée au corps de fixation **5** à l'aide du deuxième ensemble **II** d'assemblage du type écrou. Dans un exemple préféré de réalisation, cet ensemble d'assemblage **II** est un écrou **33** du type de celui décrit dans la demande de brevet **WO 98/41 159.**

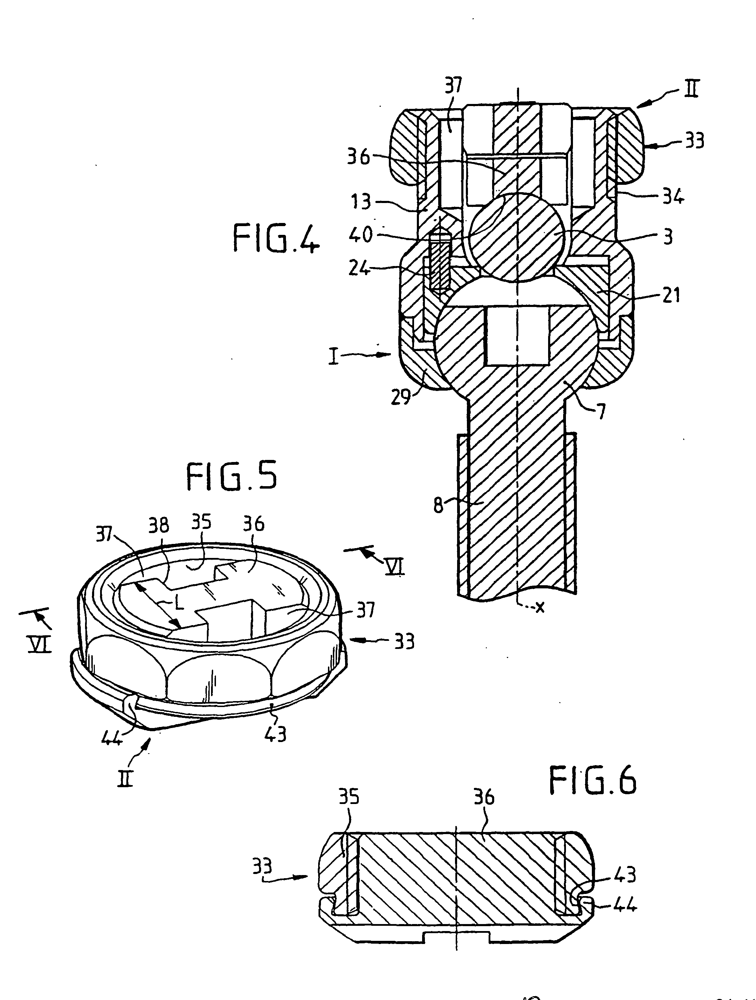

Selon cet exemple préféré de réalisation illustré plus particulièrement aux **fig. 4** à **6,** les branches latérales **13** possèdent des parois extérieures **34** qui s'inscrivent dans un cercle et sont filetées pour recevoir l'écrou fileté 33 possédant de manière classique, une section externe polygonale pour permettre sa préhension par un outil approprié. L'écrou 33 présente un taraudage 35 destiné à être vissé sur les parois filetées 34 des branches latérales 13.

L'écrou 33 est équipé d'un patin 36 s'étendant diamétralement à l'intérieur du taraudage 35 et monté libre en rotation sur l'écrou 33 pour venir en appui sur la tige de liaison 3, en vue d'assurer son blocage par serrage, entre ledit patin 36 et la bague de positionnement 21. Le patin 36 possède une largeur L adaptée pour délimiter, de part et d'autre, deux dégagements 37 permettant le passage chacun, d'une branche latérale 13 du corps de fixation 5. De préférence, les dégagements 37 permettent également l'insertion de deux broches d'un outil non représenté de préhension de l'écrou 33. Le positionnement des broches de l'outil de préhension peut être assuré par leur engagement dans des encoches 38 aménagées sur les bords latéraux du patin 36. Pour permettre le guidage des broches de l'outil de préhension et, par suite, une indexation en aveugle du patin 36 entre les branches latérales 13, les parois internes de chaque branche latérale 13 comportent une rainure longitudinale 39 s'étendant de l'extrémité libre des branches 13 jusqu'à la tête de fixation 11. Selon une caractéristique avantageuse, le patin 35 comporte une surface transversale interne 40 concave et congruente de la surface supérieure de la tige de liaison 3.

Selon une caractéristique de réalisation illustrée aux **fig. 5** et **6**, le patin 36 est monté par encliquetage sur l'écrou 33. Comme cela ressort des **fig. 5** et **6,** l'écrou 33 comporte une rainure périphérique 43 aménagée à la base de l'écrou et destinée à recevoir une nervure 44 prolongeant de part et d'autre le patin 36 pour venir s'engager, après déformation élastique, dans la rainure 43.

La mise en oeuvre de l'implant **1** selon l'invention, composé de deux ensembles **I, II,** découle directement de la description qui précède.

L'ensemble **I** qui est dépourvu de l'ensemble **II,** est d'abord utilisé en vue d'assurer l'implantation de la vis d'ancrage **2** dans une vertèbre déterminée. Ensuite, la tige de liaison 3 est placée pour être introduite entre les branches latérales 13 du corps de fixation 5. Compte tenu de la libre rotation relative entre la vis d'ancrage 2 et le corps de fixation 5, il se produit un auto-positionnement de la tige de liaison 3 à l'intérieur du canal de réception 14 du corps 5.

L'écrou 33 est ensuite vissé sur les parois extérieures 34 des branches latérales 13 avec le patin 36 engagé entre les branches 13. Le vissage de l'écrou 33 entraîne le déplacement du patin 36 qui vient en appui sur la tige de liaison 3. La poursuite de l'opération de vissage conduit au déplacement linéaire limité de la bague de positionnement 21 qui exerce un effort sur la tête 7 de la vis d'ancrage 2. Un tel vissage de l'écrou 33 conduit au blocage par serrage, d'une part, de la vis d'ancrage 2 entre la bague de positionnement 21 et la coupelle d'obturation 29 et, d'autre part, de la tige de liaison 3 entre le patin 36 et la bague de positionnement 21. Il est à noter que le serrage de l'écrou 33 sur les parois extérieures 34 des branches latérales 13 évite leur écartement lors de l'application de l'effort d'appui du patin 36 sur la tige de liaison 3. Un tel assemblage permet d'obtenir une surface de contact importante du patin **36** sur la tige de liaison **3** entraînant un blocage efficace et durable de la tige de liaison 3 par rapport au corps de fixation 5.

L'invention n'est pas limitée aux exemples décrits et représentés car diverses modifications peuvent y être apportées sans sortir de son cadre comme défini par les revendications.

## Revendications

1. Implant pour dispositif d'ostéosynthèse notamment du rachis, comportant :
- un premier ensemble (**I**) comportant :
• un corps de fixation (**5**) pour une tige de liaison (**3**), ledit corps étant aménagé pour présenter un logement de réception (**6**) pour une tête (7) d'une vis d'ancrage (**2**), afin de déterminer une articulation sphérique entre la vis d'ancrage et le corps de fixation,
• une bague de positionnement (21) destinée à être interposée entre la tête (**7**) de la vis d'ancrage et la tige de liaison (**3**),
- et un deuxième ensemble (**II**) comportant un système du type écrou (**33**), pour l'assemblage de la tige de liaison (**3**) sur le corps de fixation (**5**),
**caractérisé en ce que** :
- le premier ensemble (**I**) comporte la bague de positionnement (**21**) qui est guidée en déplacement linaire limitée dans le corps de fixation (**5**) pour autoriser, en l'absence de la tige de liaison (**3**), une libre rotation entre le corps et la vis d'ancrage,
- le deuxième ensemble (**II**) comporte le système du type écrou (**33**) qui est adapté pour permettre lors de son vissage sur le corps, l'appui sur la tige de liaison (3) et le déplacement linéaire de la bague de positionnement (21), en vue d'assurer le blocage par serrage, d'une part, de la tige de liaison (3) entre ledit système et la bague de positionnement (21) et, d'autre part, de la vis d'ancrage (2) entre la bague de positionnement (21) et le corps de fixation (5).

2. Implant selon la revendication 1, **caractérisé en ce que** :
- le corps de fixation (**5**) comprend deux branches latérales (**13**) délimitant entre elles un canal (**14**) s'ouvrant de part et d'autre du corps pour recevoir la tige de liaison (**3**), les branches latérales (**13**) présentant des parois extérieures filetées (**34**),
- et **en ce que** le système d'assemblage (**II**) comporte un écrou (**33**) adapté pour être vissé sur les parois extérieures filetées (**34**) des branches latérales (13), l'écrou (33) étant équipé dans sa zone
- diamétrale, d'un patin (36) monté libre en rotation et destiné à venir en appui sur la tige de liaison (3) pour assurer son blocage par serrage, entre ledit patin (36) et la bague de positionnement (21).

3. Implant selon la revendication 1, **caractérisé en ce que** la bague de positionnement (21) présente une surface concave (28) congruente de la tige de liaison (3) et se trouve guidée en coulissement, de manière que la surface concave délimite en partie le canal de réception (14) de la tige de liaison, en vue d'obtenir un positionnement automatique de la tige de liaison (3) entre les branches latérales (**13**) et sur la bague de positionnement (**21**).

4. Implant selon la revendication 3, **caractérisé en ce que** la bague de positionnement (**21**) est guidée en déplacement linéaire limité par rapport au corps de fixation (5) par l'intermédiaire d'un pion de guidage (24) coopérant avec un alésage complémentaire.

5. Implant selon la revendication 2, 3 ou 4, **caractérisé en ce que** la bague de positionnement (21) présente une ouverture de passage (27) s'ouvrant entre les branches latérales (13) et sur la tête (7) de la vis d'ancrage dans laquelle est aménagé un trou borgne (9) adapté pour recevoir un outil de vissage traversant l'ouverture de passage (27).

6. Implant selon la revendication 1, 2 ou 4, **caractérisé en ce que** le corps de fixation (5) comporte :
- une tête de fixation (11) à partir de laquelle s'élèvent les deux branches latérales (13) et dans laquelle est aménagée une cavité (12) s'ouvrant d'un côté entre les branches latérales (13) et du côté opposé,
- la bague de positionnement (21) montée en déplacement limité à l'intérieur de la cavité (12) avec sa surface de réception de la tige de liaison s'ouvrant entre les deux branches latérales,
- la tête (7) de la vis d'ancrage (2) montée, au moins en partie, à l'intérieur de la cavité (12), de sorte que la bague de positionnement (21) se trouve interposée entre ladite tête (7) et le corps (5),
- et une coupelle d'obturation (29) fixée sur le corps de fixation (5) du côté de sa face interne pour fermer la cavité (12) en étant traversée par la vis d'ancrage.

7. Implant selon la revendication 1 ou 5, **caractérisé en ce que** la bague de positionnement (**21**) et la coupelle d'obturation (**29**) présentent des alésages (**22**, **30**) en partie sphérique, afin de délimiter le logement de réception (**6**) pour la tête (**7**) de la vis d'ancrage.

8. Implant selon la revendication 1, **caractérisé en ce que** l'écrou (**33**) comporte un patin (**36**) dont la largeur est adaptée pour délimiter, de part et d'autre, deux dégagements (**37**) pour, d'une part, l'insertion de deux broches d'un outil de préhension de l'écrou et, d'autre part, le passage des branches latérales (**13**) du corps de fixation, en vue de permettre le coulissement dudit patin (**36**) entre les branches latérales (**13**).

9. Implant selon les revendications 2 et 8, **caractérisé en ce que** le corps de fixation (**5**) comporte deux rainures (**39**) aménagées en vis-à-vis sur les parois internes des branches latérales pour assurer, après montage de la tige de liaison (**3**), le guidage des broches de l'outil sur le corps de fixation et une indexation en aveugle du patin (**36**) entre les branches latérales (**13**).

10. Implant selon la revendication 9, **caractérisé en ce que** l'écrou (**33**) comporte un patin (**36**) sur les bords latéraux desquels sont aménagées deux encoches (**38**) s'ouvrant dans les dégagements (**37**) et destinées à l'introduction et au positionnement des broches de l'outil de préhension.

11. Implant selon la revendication 8, **caractérisé en ce que** l'écrou (**33**) comporte des moyens (**43, 44**) de montage par encliquetage du patin qui, après montage, est libre en rotation par rapport à l'écrou.

## Patentansprüche

1. Implantat für eine Osteosynthesevorrichtung, insbesondere der Wirbelsäule, umfassend:
- einen ersten Aufbau (I), umfassend:
• einen Fixierungskörper (5) für eine Verbindungsstange (3), wobei der Körper angebracht ist, um einen Aufnahmeort (6) für einen Kopf (7) einer Ankerschraube (2) aufzuweisen, um ein Kugelgelenk zwischen der Ankerschraube und dem Fixierungskörper zu bestimmen,
• einen Positionierungsring (21), der vorgesehen ist, um zwischen dem Kopf (7) der Ankerschraube und der Verbindungsstange (3) eingeschoben zu werden,
- und einen zweiten Aufbau (II), der ein System vom Schraubenmutterntyp (33) umfaßt für die Anordnung der Verbindungsstange (3) auf dem Fixierungskörper (5),
**dadurch gekennzeichnet, daß**:
- die erste Anordnung (I) den Positionierring (21) umfaßt, der zu begrenzter linearer Bewegung in dem Fixierungskörper (5) geführt ist, um bei Abwesenheit der Verbindungsstange (3) eine freie Rotation zwischen dem Körper und der Ankerschraube zuzulassen,
- die zweite Anordnung (II) das System vom Schraubenmutterntyp (33) umfaßt, das ausgelegt ist, um bei seinen Schrauben in den Körper den Halt auf der Verbindungsstange (3) und die lineare Bewegung des Positionierrings (21) im Hinblick darauf zuzulassen, das Blockieren durch Verklemmen eines Teils der Verbindungsstange (3) zwischen dem System und dem Positionierring (21) sicherzustellen und andererseits der Ankerschraube (2) zwischen dem Positionierring (21) und dem Fixierungskörper (5).

2. Implantat nach Anspruch 1, **dadurch gekennzeichnet, daß**:
- der Fixierungskörper (5) zwei Seitenarme (13) umfaßt, die untereinander einen Kanal (14) begrenzen, der sich beiderseits des Körpers öffnet, um die Verbindungsstange (3) aufzunehmen, wobei die Seitenarme (13) Außengewindewände (34) aufweisen,
- und das Verbindungssystem (II) eine Schraubenmutter (33) umfaßt, die ausgelegt ist, um auf die Außengewindewände (34) der Seitenarme (13) geschraubt zu werden, wobei die Schraubenmutter (33) in ihrem Durchmesserbereich mit einem Schuh (36) ausgerüstet ist, der rotationsfrei angebracht und dazu vorgesehen ist, zu einem Halt auf der Verbindungsstange (3) zu kommen, um deren Blockieren durch Verklemmen zwischen dem Schuh (36) und dem Positionierring (21) sicherzustellen.

3. Implantat nach Anspruch 1, **dadurch gekennzeichnet, daß** der Positionierring (21) eine konkave Oberfläche (28) aufweist, die kongruent mit der Verbindungsstange (3) ist und sich derart schiebegeführt vorfindet, daß die konkave Oberfläche teilweise den Aufnahmekanal (14) der Verbindungsstange im Hinblick darauf begrenzt, eine automatische Positionierung der Verbindungsstange (3) zwischen den Seitenarmen (13) und dem Positionierring (21) zu erhalten.

4. Implantat nach Anspruch 3, **dadurch gekennzeichnet, daß** der Positionierring (21) in begrenzter Linearbewegung im Verhältnis zum Fixierungskörper (5) mit einem Führungsstift (24) geführt ist, der mit einer komplementären Bohrung zusammenwirkt.

5. Implantat nach einem der Ansprüche 2, 3 oder 4, **dadurch gekennzeichnet, daß** der Positionierring (21) eine Durchgangsöffnung (27) aufweist, die sich zwischen den Seitenarmen (13) und dem Kopf (7) der Ankerschraube öffnet, in welchem ein Blindloch (9) eingefügt ist, das dazu ausgelegt ist, ein Schraubwerkzeug zu empfangen, das die Durchgangsöffnung (27) durchquert.

6. Implantat nach einem der Ansprüche 1, 2 oder 4, **dadurch gekennzeichnet, daß** der Fixierungskörper (5) umfaßt:
- einen Fixierkopf (11) aus den zwei Seitenarme (13) herausragen und in dem eine Höhlung (12) eingefügt ist, die sich von einer Seite zwischen den Seitenarmen (13) und der gegenüberliegenden Seite führt,
- den Positionierring (21), angebracht mit begrenzter Bewegung in der Höhlung (12) mit ihrer Aufnahmeoberfläche der Verbindungsstange, die sich zwischen den beiden Seitenarmen öffnet,
- der Kopf (7) der Ankerschraube (2), wenigstens teilweise in der Höhlung (12) derart angebracht ist, daß der Positionierring (21) sich zwischen dem Kopf (7) und dem Körper (5) eingefügt befindet,
- und einer Verschlußkuppel (29), die auf dem Fixierkörper (5) auf der Seite seiner Innenseite fixiert ist, um die Höhlung (12) unter Durchquerung durch die Ankerschraube zu schließen.

7. Implantat nach Anspruch 1 oder 5, **dadurch gekennzeichnet, daß** der Positionierring (21) und die Verschlußkuppel (29) Bohrungen (22, 30) im kugelförmigen Teil aufweisen, um den Aufnahmeort (6) für den Kopf (7) der Ankerschraube zu begrenzen.

8. Implantat nach Anspruch 1, **dadurch gekennzeichnet, daß** die Schraubenmutter (33) eine Schuh (36) umfaßt, dessen Breite angepaßt ist, um beiderseitig zwei Freistiche (37) für einerseits den Einschub von zwei Zapfen eines Greifwerkzeugs der Schraubenmutter und andererseits den Durchgang der zwei Seitenarme (13) des Fixierungskörpers im Hinblick darauf zu begrenzen, das Verschieben des Schuhs (36) zwischen den Seitenarmen (13) zuzulassen.

9. Implantat nach den Ansprüchen 2 und 8, **dadurch gekennzeichnet, daß** der Fixierungskörper (5) zwei Vertiefungen (39) umfaßt, die gegenüber auf den Innenwänden der Seitenarme angebracht sind, um nach Montieren der Verbindungsstange (3) das Führen der Zapfen des Werkzeugs auf dem Fixierungskörper und eine Blindindizierung des Schuhs (36) zwischen den Seitenarmen (13) sicherzustellen.

10. Implantat nach Anspruch 9, **dadurch gekennzeichnet, daß** die Schraubenmutter (33) einen Schuh (36) auf den Seitenrändern umfaßt, bei denen zwei Kerben (38) angebracht sind, die sich in die Freistiche (37) öffnen und zur Einführung und Positionierung der Zapfen des Greifwerkzeuges vorgesehen sind.

11. Implantat nach Anspruch 8, **dadurch gekennzeichnet, daß** die Schraubenmutter (33) Mittel (43, 44) zum Montieren durch Einklicken des Schuhs umfaßt, der nach Montage rotationsfrei im Verhältnis zur Schraubenmutter ist.

## Claims

1. Implant for osteosynthesis device in particular of the backbone comprising:
- a first assembly (I) comprising:
• an attachment body (5) for a connection rod (3), said body being arranged so as to have a housing (6) receiving a head (7) of an anchor screw (2) to determine spherical articulation between the anchor screw and the attachment body,
• a positioning ring (21) to be interposed between the head (7) of the anchor screw and the connection rod (3), and
- a second assembly (II) comprising a system of nut type (33) for assembling connection rod (3) onto attachment body (5),
**characterized in that**:
- the first assembly (I) comprises positioning ring (21) which is guided in limited linear movement within the attachment body (5) to allow free rotation, in the absence of connection rod (3), between the body and the anchor screw,
- the second assembly (II) comprises the nut-type system (33) adapted so that when being screwed onto the body, it enables bearing on the connection rod (3) and linear movement of the positioning ring (21) to ensure the locking, by clamping, firstly of the connection rod (3) between said system and the positioning ring (21), and secondly of the anchor screw (2) between the positioning ring (21) and the attachment body (5).

2. Implant as in claim 1, **characterized in that**:
- the attachment body (5) comprises two side branches (13) delimiting between each other a channel (14) opening either side of the body to receive the connection rod (3), the side branches (13) having threaded outer walls (34),
- and **in that** the assembly system (II) comprises a nut (33) adapted for screwing onto the threaded outer walls (34) of the side branches (13), the nut (33) being equipped in its diametric zone with a shoe (36) mounted in free rotation and intended to bear upon the connection rod (3) to ensure locking of the connection rod, by clamping, between said shoe (36) and the positioning ring (21).

3. Implant as in claim 1, **characterized in that** the positioning ring (21) has a concave surface (28) congruent with the connection rod (3) and is guided by sliding so that the concave surface partly delimits the receiving channel (14) of the connection rod so as to obtain the automatic positioning of the connection rod (3) between the side branches (13) and on the positioning ring (21).

4. Implant as in claim 3, **characterized in that** the positioning ring (21) is guided in limited linear movement with respect to the attachment body (5) by a guide pin (24) cooperating with a complementary bore.

5. Implant as in claim 2, 3 or 4 **characterized in that** the positioning ring (21) has a passage opening (27) which opens between the side branches (13) and on the head (7) of the anchor screw in which a blind hole (9) is provided adapted to receive a screwing tool passing through the passage opening (27).

6. Implant as in claim 1, 2 or 4 **characterized in that** the attachment body (5) comprises:
- an attachment head (11) from which the two side branches (13) protrude and in which a cavity (12) is arranged opening on one side between the side branches (13) and on the opposite side,
- the positioning ring (21) mounted with limited movement within cavity (12) with its receiving surface for the connection rod opening between the two side branches,
- the head (7) of the anchor screw (2) mounted, at least in part, inside cavity (12) so that the positioning ring (21) lies between said head (7) and the body (5),
- and a sealing cup (29) fixed to the attachment body (5) on the side of its inner face to close the cavity (12) and through which the anchor screw passes.

7. Implant as in claim 1 or 5, **characterized in that** the positioning ring (21) and the sealing cup (29) have partly spherical bores (22, 30) to delimit the housing (6) receiving the head (7) of the anchor screw.

8. Implant as in claim 1, **characterized in that** the nut (33) comprises a shoe (36) whose width is adapted to delimit, on either side, two clearances (37) firstly for the insertion of the two grippers of a nut gripping tool and secondly for passing the side branches (13) of the attachment body to allow the sliding of said shoe (36) between the side branches (13).

9. Implant as in claims 2 and 8, **characterized in that** the attachment body (5) comprises two grooves (39) arranged opposite one another on the inner walls of the side branches so that, after connection rod (3) is mounted, they ensure the guiding of the grippers on the attachment body and the blind indexing of shoe (36) between the side branches (13).

10. Implant as in claim 9, **characterized in that** the nut (33) comprises a shoe (36) on whose side edges two notches (38) are provided opening into clearances (37) intended for inserting and positioning the grippers of the gripping tool.

11. Implant as in claim 8, **characterized in that** the nut (33) comprises means (43, 44) for mounting the shoe by snap-fitting which, after mounting, is freely rotational with respect to the nut.
